# EUROPEAN PATENT APPLICATION

(11) **EP 4 578 958 A1**
(43) Date of publication of application: **02.07.2025**
(21) Application number: 24759593.7
(22) Date of filing: 07.02.2024
(51) Int. Cl.: C12Q 1/6806, C12Q 1/6869, C40B 50/06

(54) **METHOD FOR CONSTRUCTING SINGLE CELL LIBRARY CONTAINING TISSUE LOCATION INFORMATION AND SEQUENCING METHOD**

(30) Priority: 20.02.2023 CN 202310135198
(71) Applicant: Beijing SeekGene Biosciences Co., Ltd., Beijing 102200 (CN)
(72) Inventor: HAN, Jinhuan, Beijing 102200 (CN); GUAN, Ying, Beijing 102200 (CN); ZHI, Xiaoling, Beijing 102200 (CN); ZHI, Min, Beijing 102200 (CN); SANG, Guoqin, Beijing 102200 (CN); SHI, Huanhuan, Beijing 102200 (CN); JIAO, Shaozhuo, Beijing 102200 (CN); LI, Zongwen, Beijing 102200 (CN)
(74) Representative: Dantz, Jan Henning
(86) International application number: PCT/CN2024/076779
(87) International publication number: WO 2024/174916

(57) **Abstract**

Disclosed in the present invention are a method for constructing a single cell library containing tissue location information and a sequencing method. **The** construction method comprises the following steps: S10, providing a chip, wherein the chip is provided with a plurality of sites, each site is provided with nucleic acid molecules having a known base sequence, and the nucleic acid molecules contain location information; S20, enabling a tissue slice to be tested to be in contact with the chip, so as to bind the nucleic acid molecules having the known base sequence in the chip to cells of said tissue slice to obtain a bound tissue slice; S30, dissociating the bound tissue slice to prepare a single cell suspension; S40, constructing a library for the single cell suspension by means of a high-throughput single cell library construction and sequencing platform, to obtain a transcriptome library and a spatial barcoded library; and S50, sequencing the transcriptome library and the spatial barcoded library to obtain a single cell library containing tissue location information. According to the method, spatial location information of a single cell can be tested while analyzing genetic information on a single cell level.

## Description

### CROSS-REFERENCE TO RELATED APPLICATIONS

This application claims priority to Chinese Patent Application No. 202310135198.3, filed on February 20, 2023, which is incorporated herein by reference in its entirety.

### TECHNICAL FIELD

The present disclosure relates to the technical field of gene sequencing and tissue cell sample analysis, and in particular to a method for constructing a single-cell library containing spatial information of cells in tissue and a sequencing method.

### BACKGROUND

It is well known that complex life forms are composed of many cells with specific properties. Under certain conditions, the types and quantities of nucleic acids and proteins expressed by each cell vary. Therefore, detecting nucleic acid and protein indicators at the single-cell level, as well as the microenvironment of the cells, is of great significance for biomedical research.

Currently, single-cell research technologies, including high-throughput single-cell transcriptomics, can only analyze nucleic acid information and some protein information at the single-cell level, but little is known about the spatial location of individual cells within tissues. To gain a deeper understanding of nucleic acid information at the tissue level, scientists have developed various spatial omics technologies, including FISSEQ, Slide-seq, 10×Visium, HDST, SeqFish, MerFish, and STARmap. These technologies can be roughly divided into three types based on their implementation methods: in situ hybridization, in situ capture, and in situ sequencing. In situ hybridization (SeqFish and MerFish) and in situ sequencing (FISSEQ and STARmap) technologies rely on probes designed for genes of interest, while in situ capture technologies (Slide-seq, 10× Visium, and HDST) can perform hypothesis-free experiments targeting the entire transcriptome. Therefore, in situ capture technologies are widely used in scientific research and have significant commercial value. However, the principle of in situ capture technology is to analyze RNA information at the tissue location level and then use software to simulate the cell to which each RNA belongs based on its spatial information, rather than directly analyzing at the true physical level of single cells within the tissue. Considering that random diffusion of RNA during in situ capture and the presence of multiple cell layers in slices can lead to severe distortion of the true cellular localization of RNA, it is urgent to develop a single-cell technology that can simultaneously detect the spatial information of single cells within tissues and the nucleic acids of single cells.

### SUMMARY OF THE INVENTION

A main objective of the present disclosure is to propose a method for constructing a single-cell library containing spatial information of cells in tissue. This method aims to provide a library construction method for co-analyzing the location and genetic information of individual cells within a tissue. It allows for the analysis of genetic information (such as transcriptome, ATAC, and methylation) at the true single-cell level while also capturing the spatial information of the single cell.

To achieve the above objective, the present disclosure proposes a method for constructing a single-cell library containing spatial information of cells in tissue, including the following steps:
S10: providing a chip with multiple sites, each site having nucleic acid molecules with a known base sequence, where the nucleic acid molecules contain spatial information;
S20: contacting a slice of a tissue to be tested with the chip so that the nucleic acid molecules with known base sequences in the chip bind to cells of the slice of the tissue to be tested, resulting in a bound tissue slice;
S30: dissociating the bound tissue slice to prepare a single-cell suspension;
S40: constructing a library for the single-cell suspension using a high-throughput single-cell library construction and sequencing platform to obtain a transcriptome library and a spatial tag library; and
S50: sequencing the transcriptome library and the spatial tag library to obtain the single-cell library containing spatial information of cells in tissue.

Optionally, in step S10,
nucleic acid molecules at different sites have different known base sequences, and nucleic acid molecules at the same site have the same known base sequence; and/or a spacing between sites is 100 nm to 1 mm.

Optionally, in step S10,
the chip can be a planar chip, and nucleic acid molecule clusters with spatial information are independently and relatively uniformly distributed on a surface of the chip;
the chip has micropores in which a medium is contained, and the nucleic acid molecules with spatial information are distributed on the medium in the micropores of the chip or coupled to the surface of the chip;
the chip has micropores in which a medium is contained, and the medium in the micropores of the chip includes any one of polymer beads, magnetic beads, and hydrogel beads; and/or
a material of the chip includes any one of glass, ITO glass, polymer, ITO polymer, and silicon wafer.

Optionally, in step S10,
the nucleic acid molecule with spatial information is coupled with a molecule that binds to a specific substance in the cell.

Optionally, the molecule that binds to the specific substance in the cell includes a lectin, and the specific substance in the cell is a glycoprotein;
the molecule that binds to the specific substance in the cell includes an antibody, and the specific substance in the cell is β2-microglobulin;
the molecule that binds to the specific substance in the cell includes a lipid molecule, and the specific substance in the cell is a lipid bilayer; or
the molecule that binds to the specific substance in the cell includes a nucleic acid, and the specific substance in the cell is mRNA.

Optionally, between step S10 and step S20, the method further includes: soaking the cells of the slice of the tissue to be tested with any one of fixatives selected from an aldehyde fixative solution, an acid fixative solution, an alcohol fixative solution, and a DSP crosslinker; and/or
soaking the cells of the slice of the tissue to be tested with an alcohol or a surfactant.

Optionally, in step S10, the chip is prepared by the following steps:
S01: synthesizing various nucleic acid molecules with different spatial tag sequences; and
S02: contacting the synthesized nucleic acid molecules with corresponding positions on the chip, so that the synthesized nucleic acid molecules are fixed to the corresponding positions on the chip, to obtain the chip with the nucleic acid molecules containing spatial information.

Optionally, in step S01,
the nucleic acid molecule includes a fixed sequence -a conditionally cleavable site - a spatial tag - a cell binding molecule, where the conditionally cleavable site includes any one of a disulfide modification, a dU modification, an RNA base modification, a dI modification, a DSpacer modification, an AP site modification, a photocleavable (PC) linker, and a restriction enzyme recognition sequence; and/or
the nucleic acid molecule includes a 5' coupling group and a molecular tag sequence, where the 5' coupling group includes any one of a chemical crosslinker, biotin, avidin, and streptavidin.

Optionally, in step S01,
the nucleic acid molecule includes a 5' coupling group and a molecular tag sequence, where the 5' coupling group includes any one of a chemical crosslinker, biotin, avidin, and streptavidin; and the chemical crosslinker includes any one of cystamine, glutaraldehyde, S,S-dimethyl sulfoximine, N-hydroxysulfosuccinimide crosslinker BS3, formaldehyde, carbodiimide, SMCC, sulfo-SMCC, vinylsilane, N,N'-diallyltartaridiamide, and N,N'-bis(acryloyl)cystamine.

Optionally, in step S02,
a method for fixing the synthesized nucleic acid molecules to the corresponding positions on the chip includes either of an in situ contact method and a random fixation method.

The present disclosure further provides a sequencing method, including the following steps:
constructing a single-cell library using the method for constructing a single-cell library containing spatial information of cells in tissue, to obtain a transcriptome library and a spatial tag library;
sequencing the obtained transcriptome library and the spatial tag library to obtain single-cell transcriptome data and spatial tag library data; and
mapping spatial information and genetic information of the nucleic acid molecules with known base sequences to the tissue to be tested to obtain spatial information and genetic information of single cells in the tissue to be tested.

The method for constructing a single-cell library containing spatial information of cells in tissue includes the following steps:
S10: providing a chip with multiple sites, each site having nucleic acid molecules with a known base sequence, where the nucleic acid molecules contain spatial information;
S20: contacting a slice of a tissue to be tested with the chip so that the nucleic acid molecules with known base sequences in the chip bind to cells of the slice of the tissue to be tested, resulting in a bound tissue slice;
S30: dissociating the bound tissue slice to prepare a single-cell suspension;
S40: constructing a library for the single-cell suspension using a high-throughput single-cell library construction and sequencing platform to obtain a transcriptome library and a spatial tag library; and
S50: sequencing the transcriptome library and the spatial tag library to obtain the single-cell library containing spatial information of cells in tissue.

The present invention provides a method for constructing a single-cell library containing spatial information of cells in tissue. By contacting the nucleic acid chips having spatial information with slices of a tissue to be tested, the spatial information can be released into the tissue cells, so that during the subsequent dissociation of the tissue cells, the spatial information of the tissue can be read, thereby enabling the analysis of genetic information at the single-cell level while also obtaining the spatial information of the single cells.

### BRIEF DESCRIPTION OF THE DRAWINGS

To more clearly illustrate the technical solutions in the embodiments of the present disclosure or the prior art, a brief introduction to the accompanying drawings required for the description of the embodiments or the prior art is provided below. It is evident that the accompanying drawings described below are merely some embodiments of the present disclosure. For a person of ordinary skill in the art, other drawings can be obtained based on the structures shown in these drawings without creative efforts.
FIG. 1 is a schematic diagram of the principle of single-cell sequencing with spatial information when using non-nucleic acid molecules as the molecules that can bind to the cells in the tissue according to an embodiment of the present disclosure;
FIG. 2 is a schematic diagram of the principle of single-cell sequencing with spatial information when using nucleic acids as the molecules that can bind to the cells in the tissue according to an embodiment of the present disclosure;
FIG. 3 is a schematic structural diagram of the planar spatial nucleic acid chip and the spatial nucleic acid chip having a microporous structure according to an embodiment of the present disclosure;
FIG. 4 is a schematic structural diagram of the nucleic acid molecule with spatial information on the nucleic acid chip according to an embodiment of the present disclosure;
FIG. 5 is a schematic diagram of the principle of constructing the spatial nucleic acid chip using the in situ contact method according to an embodiment of the present disclosure;
FIG. 6 is a schematic diagram of the principle of constructing the spatial nucleic acid chip using the random fixation method after ligation between the medium and spatial information according to an embodiment of the present disclosure;
FIG. 7 is a structural diagram of the single-cell library containing spatial tags according to an embodiment of the present disclosure;
FIG. 8 is a diagram of quality control of the spatial tag library according to an embodiment of the present disclosure; and
FIG. 9 is a diagram of quality control of the single-cell library containing spatial tags according to an embodiment of the present disclosure.

The realization of the purpose, functional characteristics, and advantages of this invention will be further explained in conjunction with the examples and with reference to the drawings.

### DETAILED DESCRIPTION

To make the objectives, technical solutions, and advantages of embodiments of the present disclosure clearer, the technical solutions in the embodiments of the present disclosure will be described clearly and completely below. For an embodiment for which a detailed condition is not specified, the embodiment is conducted under a regular condition or a condition recommended by a manufacturer. Any reagent or instrument used for which no manufacturer is specified is a regular commercially available product. In addition, "and/or" in this specification includes three parallel schemes. For example, "A and/or B" includes a scheme A, or a scheme B, or both the scheme A and the scheme B. In addition, technical solutions in the embodiments may be combined with each other, provided that they can be implemented by a person of ordinary skill in the art. When the combination of technical solutions is contradictory or unimplementable, it should not be considered that the combination of technical solutions exists or falls within the claimed protection scope of the present disclosure. All other embodiments obtained by a person of ordinary skill in the art on the basis of the embodiments of the present disclosure without creative effort shall fall within the scope of protection of the present disclosure.

It is well known that complex life forms are composed of many cells with specific properties. Under certain conditions, the types and quantities of nucleic acids and proteins expressed by each cell vary. Therefore, detecting nucleic acid and protein indicators at the single-cell level, as well as the microenvironment of the cells, is of great significance for biomedical research.

Currently, single-cell research technologies, including high-throughput single-cell transcriptomics, can only analyze nucleic acid information and some protein information at the single-cell level, but little is known about the spatial location of individual cells within tissues. To gain a deeper understanding of nucleic acid information at the tissue level, scientists have developed various spatial omics technologies, including FISSEQ, Slide-seq, 10×Visium, HDST, SeqFish, MerFish, and STARmap. These technologies can be roughly divided into three types based on their implementation methods: in situ hybridization, in situ capture, and in situ sequencing. In situ hybridization (SeqFish and MerFish) and in situ sequencing (FISSEQ and STARmap) technologies rely on probes designed for genes of interest, while in situ capture technologies (Slide-seq, 10× Visium, and HDST) can perform hypothesis-free experiments targeting the entire transcriptome. Therefore, in situ capture technologies are widely used in scientific research and have significant commercial value. However, the principle of in situ capture technology is to analyze RNA information at the tissue location level and then use software to simulate the cell to which each RNA belongs based on its spatial information, rather than directly analyzing at the true physical level of single cells within the tissue. Considering that random diffusion of RNA during in situ capture and the presence of multiple cell layers in slices can lead to severe distortion of the true cellular localization of RNA, it is urgent to develop a single-cell technology that can simultaneously detect the spatial information of single cells within tissues and the nucleic acids of single cells.

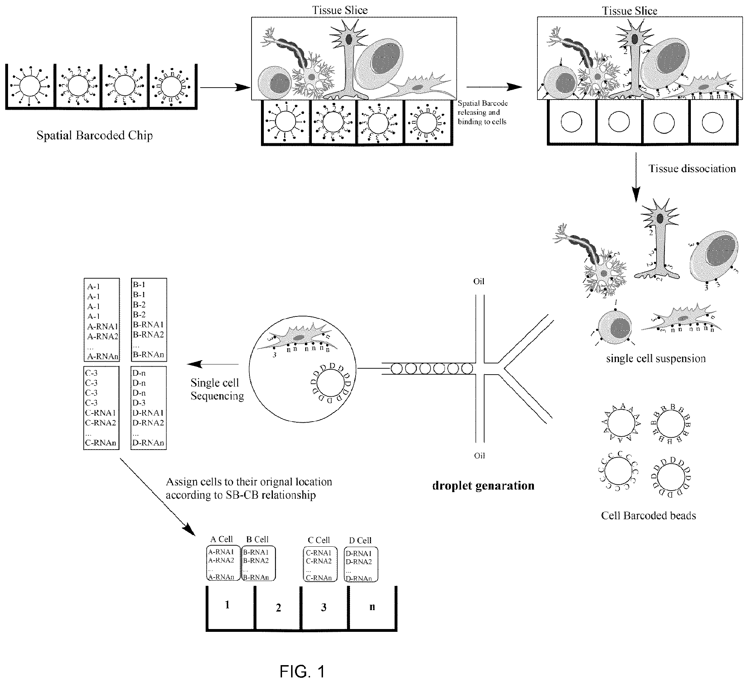

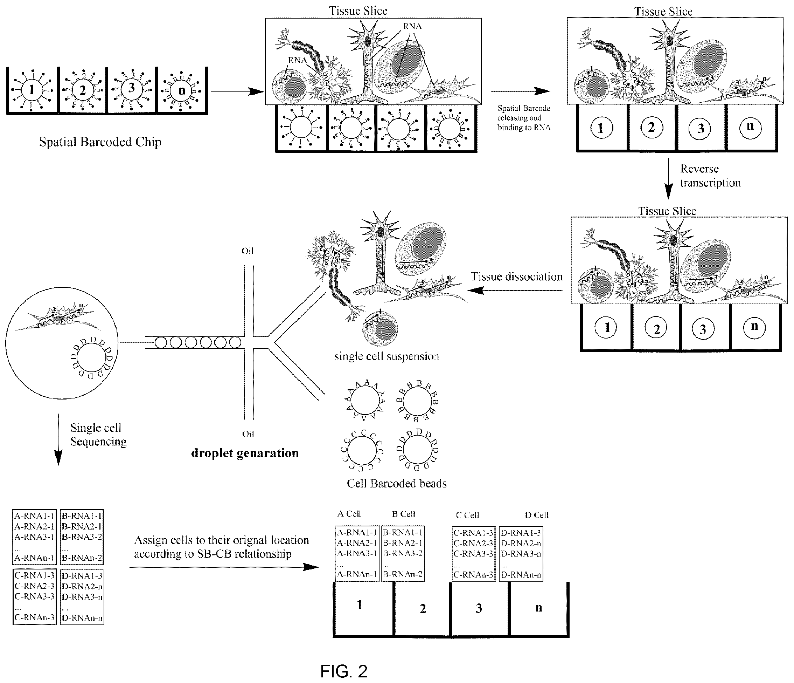

In view of this, as shown in FIG. 1 and FIG. 2, the present disclosure is to propose a method for constructing a single-cell library containing spatial information of cells in tissue. This method aims to provide a library construction method for co-analyzing the location and genetic information of individual cells within a tissue.

The present disclosure provides a first embodiment of a method for constructing a single-cell library containing spatial information of cells in tissue, including the following steps:
S10: providing a chip with multiple sites, each site having nucleic acid molecules with a known base sequence, where the nucleic acid molecules contain spatial information;
S20: contacting a slice of a tissue to be tested with the chip so that the nucleic acid molecules with known base sequences in the chip bind to cells of the slice of the tissue to be tested, resulting in a bound tissue slice;
S30: dissociating the bound tissue slice to prepare a single-cell suspension;
S40: constructing a library for the single-cell suspension using a high-throughput single-cell library construction and sequencing platform to obtain a transcriptome library and a spatial tag library; and
S50: sequencing the transcriptome library and the spatial tag library to obtain the single-cell library containing spatial information of cells in tissue.

In the technical solution of the present disclosure, by contacting the nucleic acid chips having spatial information with slices of a tissue to be tested, the spatial information can be released into the tissue cells, so that during the subsequent dissociation of the tissue cells, the spatial information of the tissue can be read, thereby enabling the analysis of genetic information at the single-cell level while also obtaining the spatial information of the single cells.

Further, in step S10, nucleic acid molecules at different sites have different known base sequences, and nucleic acid molecules at the same site have the same known base sequence. This facilitates quick and accurate determination of the spatial information of the cells in the tissue to be tested in a subsequent process.

Further, in step S10, the spacing between sites ranges from 100 nm to 1 mm, particularly from 1 µm to 100 µm, or more particularly 5 µm to 50 µm, and preferably, 5 µm to 10 µm. This ensures that each tissue cell to be tested can bind to the nucleic acid molecules with spatial information.

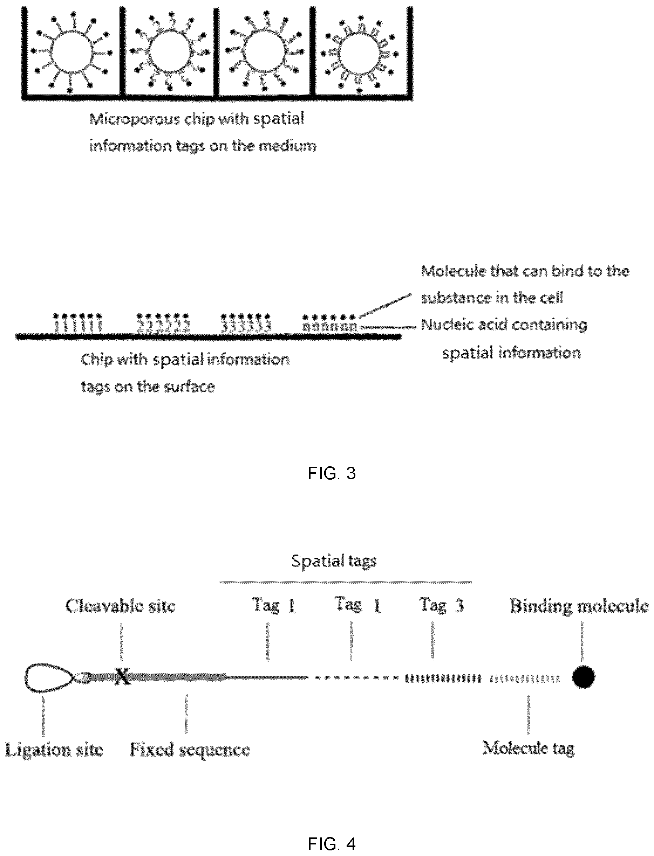

Further, as shown in FIG. 3, in step S10, the chip is planar, and the nucleic acid molecules with spatial information are fixed onto the surface of the chip in relatively independent clusters. This setup allows the nucleic acid molecules on the chip to better contact the cells of the tissue to be tested, facilitating binding.

Further, as shown in FIG. 3, in step S10, the chip has micropores in which a medium is contained, and the nucleic acid molecules with spatial information are distributed on the medium in the micropores of the chip or coupled to the surface of the chip. This setup facilitates the distribution of nucleic acid molecules on the chip and their binding with the cells in the tissue to be tested.

Further, in step S10, the chip has micropores in which a medium is contained, and the medium in the micropores of the chip includes any one of polymer beads, magnetic beads, and hydrogel beads. The design of the beads facilitates the loading of the medium into the pores at the sites. The medium can be either cleavable or non-cleavable.

Further, in step S10, the material of the chip includes any one of glass, ITO glass, polymer, ITO polymer, and silicon wafer. Different materials can be selected based on needs.

Further, in step S10, the nucleic acid molecules with spatial information are coupled with molecules that bind to a specific substance in the cell. This facilitates the binding of the nucleic acid molecules with the spatial information to the cells in the tissue to be tested.

In some embodiments of the present disclosure, in step S10, the molecules that bind to the specific substance in the cell include a lectin, and the specific substance in the cell is a glycoprotein. By specifically binding the lectin to the glycoprotein, the nucleic acids with spatial information on the chip can be bound to the cells in the tissue to be tested, thereby tagging the location of the cells of the tissue to be tested.

In some embodiments of the present disclosure, in step S10, the molecules that bind to the specific substance in the cell include an antibody, and the specific substance in the cell is β2-microglobulin. By specifically binding the antibody to β2-microglobulin, the nucleic acids with spatial information on the chip can be bound to the cells in the tissue to be tested, thereby tagging the location of the cells of the tissue to be tested.

In some embodiments of the present disclosure, in step S10, the molecules that bind to the specific substance in the cell include a lipid molecule, and the specific substance in the cell is a lipid bilayer. By specifically binding the lipid molecule to the lipid bilayer, the nucleic acids with spatial information on the chip can be bound to the cells in the tissue to be tested, thereby tagging the location of the cells of the tissue to be tested.

In some embodiments of the present disclosure, in step S10, the molecules that bind to the specific substance in the cell include a nucleic acid, and the specific substance in the cell is mRNA. By using nucleic acids such as oligo-dT, which can hybridize with the polyA tails of all mRNA, the nucleic acids with spatial information on the chip can be bound to the cells in the tissue to be tested, thereby tagging the location of the cells of the tissue to be tested.

Further, between step S10 and step S20, the method further includes: soaking the cells of the slice of the tissue to be tested with any one of fixatives selected from an aldehyde fixative solution, an acid fixative solution, an alcohol fixative solution, and a DSP crosslinker. The cells are fixed and immobile, thereby more effectively binding nucleic acid molecules to the cells adjacent to the tissue to be tested.

Further, between step S10 and step S20, the method further includes: permeabilizing the cells of the slice of the tissue to be tested. The permeabilization method includes soaking cells of the slice of the tissue to be tested in an alcohol or a surfactant. The permeabilization disrupts the integrity of the cell membrane or nuclear membrane, thereby more effectively binding nucleic acid molecules to the cells adjacent to the tissue to be tested.

It should be noted that if the molecules that can bind to the cells are nucleic acid molecules oligo-dT, a reverse transcription reaction can be further performed in step S20 to increase the stability of the spatial information binding to the cells.

In addition, in step S30, the tissue slice is dissociated to prepare a single-cell suspension, and the dissociation process does not destroy the binding relationship between the nucleic acid containing spatial information and the cells.

A high-throughput single-cell library construction and sequencing platform is used to construct a library for the single-cell suspension. Different structures of nucleic acid molecules containing spatial information and tissue fixation solutions require single-cell library construction platforms with different principles. Table 1 exemplarily shows the single-cell library construction platforms corresponding to different nucleic acid structures when using dithiobis(succinimidyl propionate) (DSP) to fix the tissue. The single-cell library construction platforms are divided into 3' single-cell transcriptome and 5' single-cell transcriptome according to different principles. When the end of the spatial nucleic acid molecule is a polyA tail (spatial tag structure-1), a mature 3' library construction kit such as SeekOne^{®} 3' single-cell transcriptome kit or 10×Genomics 3' single-cell kit can be used with Biolegend HashTagTM experiment procedure solution, to obtain a spatial tag library and a single-cell 3' transcriptome library, respectively. When the end of the spatial nucleic acid molecule is a base sequence CCCATATAAGAAA complementary to the template switching sequence in the 5' kit (spatial tag structure-2), a mature 5' library construction kit such as SeekOne^{®} 5' single-cell transcriptome kit or 10×Genomics 5' single-cell kit V2 can be used with Biolegend TotalSeqTM-C experiment procedure solution, to obtain a spatial tag library and a single-cell 5' transcriptome library respectively. When the end of the spatial nucleic acid molecule is oligo-dT30 rather than other cell-binding molecules (spatial tag structure-3), a mature 5' library construction kit such as SeekOne^{®} 5' single-cell transcriptome kit or 10×Genomics 5' single-cell kit V2 can be used to directly construct a 5' single-cell library. It only requires adding the Rd2 SeqPrimer amplification primer in the cDNA amplification step and sorting out the 300-800bp cDNA amplification product for further index PCR to obtain an additional spatial tag library

Further, in step S10, the chip is prepared through the following steps:
S01: synthesizing various nucleic acid molecules with different spatial tag sequences; and
S02: contacting the synthesized nucleic acid molecules with corresponding positions on the chip, so that the synthesized nucleic acid molecules are fixed to the corresponding positions on the chip, to obtain the chip with the nucleic acid molecules containing spatial information.

The nucleic acid molecules with different spatial tag sequences are synthesized first, and then the nucleic acid molecules with different spatial tag sequences are brought into contact with the corresponding positions on the chip, so that the spatial information at the sites on the chip is clear, thereby facilitating the tracking of the spatial information of the cells in the tissue to be tested.

Further, as shown in FIG. 4, in step S01, the nucleic acid molecule includes a fixed sequence -a conditionally cleavable site - a spatial tag - a cell binding molecule, where the conditionally cleavable site includes any one of a disulfide modification, a dU modification, an RNA base modification, a dI modification, a DSpacer modification, an AP site modification, a photocleavable (PC) linker, and a restriction enzyme recognition sequence. This setting can facilitate breakage.

Further, in step S01, the nucleic acid molecule includes a 5' coupling group and a molecular tag sequence, where the 5' coupling group includes any one of a chemical crosslinker, biotin, avidin, and streptavidin. The 5' coupling group can react with a surface of a chip matrix or the medium in the micropores of the chip to be fixed thereto.

Further, in step S01, the nucleic acid molecule includes a 5' coupling group and a molecular tag sequence, where the 5' coupling group includes any one of a chemical crosslinker, biotin, avidin, and streptavidin; and the chemical crosslinker includes any one of cystamine, glutaraldehyde, S,S-dimethyl sulfoximine, N-hydroxysulfosuccinimide crosslinker BS3, formaldehyde, carbodiimide, SMCC, sulfo-SMCC, vinylsilane, N,N'-diallyltartaridiamide, and N,N'-bis(acryloyl)cystamine.

It should be noted that in step S01, the spatial tag can be directly coupled to the chip or the medium in the micropores after a one-time synthesis, or can be coupled to the chip or the medium in the micropores in a segmented manner using a split-pool scheme; or spatial tags can be generated using in-situ amplification technology. The molecule that can bind to the substance in the cell can be synthesized directly at the 3' end of the spatial tag, or it can be ligated to the 3' end of the spatial tag sequence after the spatial nucleic acid chip is made. The ligation method can be the chemical cross-linkers mentioned above, or it can be biotin, avidin, and enzyme-mediated bioconjugation. Nucleic acid molecules with spatial information can incorporate nucleic acid modifications well-known in the field, including but not limited to one or more of amino, phosphate, alkynyl, azide, thiol, disulfide, olefin, biotin, azobenzene, methyl, spacer, photo-cleavable groups, dI, dU, LNA, XNA, ribonucleic acid bases, and dideoxyribonucleic acid bases.

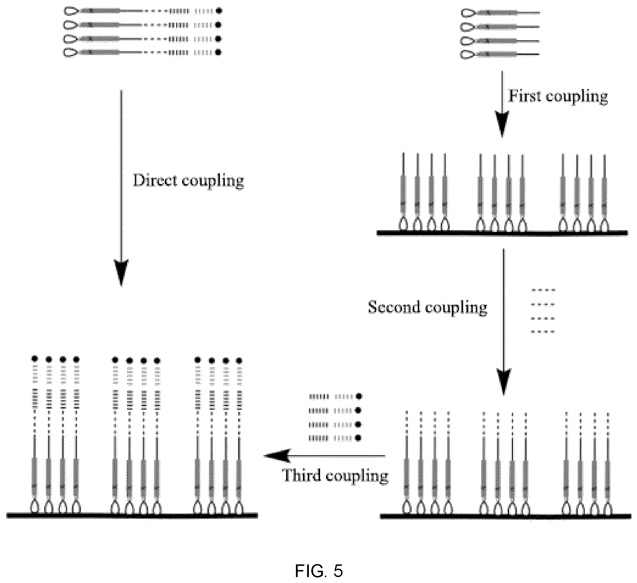

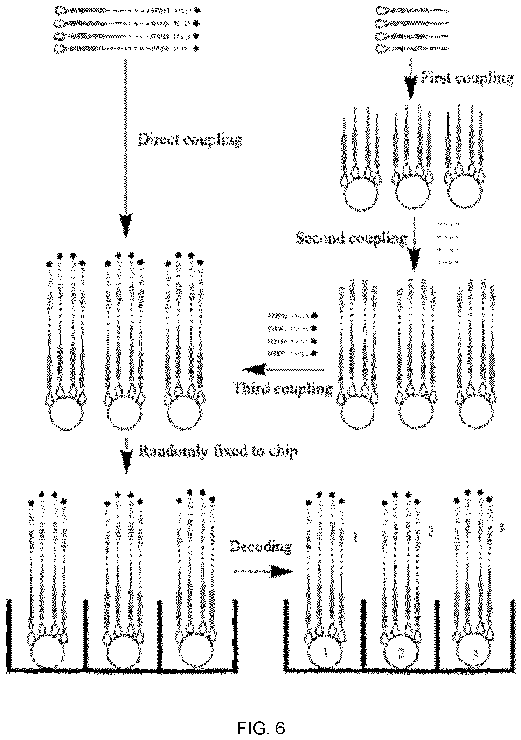

Further, in step S02, the method for fixing the synthesized nucleic acid molecules to the corresponding locations on the chip includes either an in situ contact method or a random fixation method. Specifically, there are two solutions for the contact between the nucleic acid molecule with the spatial information and the chip: the in situ contact method (FIG. 5) and the random fixation method after ligation between the medium and the spatial information (FIG. 6). As shown in FIG. 5, the in situ contact method includes: using inkjet equipment to contact and connect nucleic acid molecules with known position sequences to different positions on the chip matrix. Nucleic acid molecules can be ligated to the chip surface or micropores once or multiple times to form a nucleic acid chip with specific chip positions corresponding to nucleic acid position sequences. The same position on the chip is ligated to nucleic acid molecules with the same spatial tag, and different positions are ligated to nucleic acid molecules with different spatial tags; nucleic acid molecules can be directly coupled to the chip matrix surface or ligated to the medium in the chip micropores; the inkjet equipment can include piezoelectric ceramic instruments such as jetlab4 or multi-channel inkjet equipment such as Mercury microarrayer. As shown in Figure 6, the random fixation method after ligation between the medium and the spatial information includes: first preparing a medium with different spatial information, and then randomly contacting the medium with different positions on the chip to obtain a position chip. This medium can be polymer microspheres such as magnetic beads or hydrogels, with the same spatial information molecules coupled on the same microsphere, and different spatial information molecules coupled on different microspheres; the combination of spatial information and microspheres can be a one-step combination or a multi-step connection; it can be synthesized first and then ligated, or the spatial information molecules can be synthesized directly on the medium; after the medium with spatial information randomly contacts the chip to form a nucleic acid chip, decoding is needed to determine the specific position nucleic acid sequence corresponding to the medium at each position. The random fixation method also includes clustering technology after randomly fixing single nucleic acid molecules.

It should be noted that the spatial tag structure of the nucleic acid molecules can be Rd2 seqPrimer-spatial tag-dA30-binding group (BG), using the 3' single-cell library construction method, with the specific sequence: GTGACTGGAGTTCAGACGTGTGCTCTTCCGATCTnnnnnnnnnnnnnnnnnnnnnnnnn nnnNNNNNNNNAAAAAAAAAAAAAAAAAAAAAAAAAAAAAAAA-ConA. The spatial tag structure of the nucleic acid molecules can also be Rd2 seqPrimer-spatial tag-TSO-binding group, using the 5' single-cell library construction, with the specific sequence:
CGGAGATGTGTATAAGAGACAGnnnnnnnnnnnnnnnnnnnnnnnnnnnnNNNNNNNNC CCATATAAGAAA-ConA. The spatial tag structure of the nucleic acid molecules can also be a fixed nucleic acid sequence-spatial tag-dT30 (binding group), using the 5' single-cell library construction, with the specific sequence: GTGACTGGAGTTCAGACGTGTGCTCTTCCGATCTnnnnnnnnnnnnnnnnnnnnnnnnn nnnNNNNNNNNTTTTTTTTTTTTTTTTTTTTTTTTTTTTTT. Alternatively, the spatial tag structure of the nucleic acid molecule is fixed nucleic acid sequence-spatial tag-dT30 (binding group), using the 5' single-cell library construction, with the specific sequence:
CTCTTTTTTTTUCGTCCGTCGTTGCTCGjjjjjjjjACTGGTGAjjjjjjjjGGTAGTGACAjjjjjjjj NNNNNNNNAGACGCTTTTTTTTTTTTTTTTTTTTTTTTTTTTTT. Herein, nnnnnnnnnnnnnnnnnnnnnnnnnnnn and jjjjjjjj represent all or part of the spatial tag sequence, and NNNNNNNN represents a random molecular tag sequence.

The present disclosure further provides a sequencing method, including the following steps:
constructing a single-cell library using the method for constructing a single-cell library containing spatial information of cells in tissue, to obtain a transcriptome library and a spatial tag library;
sequencing the obtained transcriptome library and the spatial tag library to obtain single-cell transcriptome data and spatial tag library data; and
mapping spatial information and genetic information of the nucleic acid molecules with known base sequences to the tissue to be tested to obtain spatial information and genetic information of single cells in the tissue to be tested.

In particular, during sequencing, the obtained transcriptome library and spatial tag library are sequenced separately. The sequencing can be performed using an Illumina sequencer or a Beijing Genomics Institution's (BGI) sequencer, and any sequencing platform compatible with the sequencing adapter. Further, a person of ordinary skill in the art can modify the primer sequence in front of the spatial spatial tag according to the requirements of different sequencing platforms to apply to different sequencing platforms, without affecting the technical protection scope of the present disclosure.

In particular, during data analysis, the single-cell transcriptome data generated from sequencing is analyzed according to standard analytical procedures. Firstly, the estimated number of cells and corresponding cell tags can be obtained based on the number of UMIs detected for all cell tags. Further, the type and physiological state of each cell are determined based on the specific RNA expression corresponding to each cell tag. Secondly, the spatial spatial tag information corresponding to all cell tags is obtained by analyzing the spatial tag library data, each detected cell tag can correspond to at least one spatial spatial tag, and the cell can be mapped to the original position on the chip based on the spatial spatial tag. If the detected cell tag corresponds to multiple spatial spatial tags, the UMIs of all spatial tags need to be counted, sorted, and then deconvolved, to determine the true location.

The sequencing method provided in the present disclosure has all the technical solutions of the method for constructing a single-cell library containing spatial information of cells in tissue, and therefore, has all the beneficial effects of the method for constructing a single-cell library containing spatial information of cells in tissue. Details will not be repeated in the present disclosure.

The technical solution in the present disclosure is further described in detail with reference to the specific examples and accompanying drawings. It should be understood that the following examples are used only to explain the present disclosure, but are not used to limit the present disclosure.

### Example 1 Preparation of spatial nucleic acid chip

1.1. Nucleic acid-tagged magnetic beads were prepared using a conventional method.

1.2. In this step, the technical method of "dispersion-merger-dispersion" was used to prepare various nucleic acid-taged spatial barcoded beads (SBB). The detailed preparation method referred to patents CN 114096678 A and CN202080065556.2. The magnetic beads were PS carboxyl magnetic beads with a diameter of 5 µm. The SBB sequence contained dU modifications, which could be cleaved by USER enzyme to form nucleic acid molecules with a 5'P structure. The specific sequence was as follows:

5'NH2-CTCTTTTTTT-U-CGTCCGTCGTTGCTCGjjjjjjjACTGGTGAjjjjjjj
GGTAGTGACAjjjjjjjNNNNNNNNAGACGCTTTTTTTTTTTTTTTT-3'.

1.3. Loading of the SBBs into the micropores of the chip.

1.3.1. Preparation of microporous chips: custom glass microporous chips with a pore diameter and depth of 7.5 µm and a pore wall thickness of 2.5 µm were prepared. The chip size was consistent with a slide (75 mm × 25 mm), with the microporous area located at the center (10 mm × 10 mm) containing 1 million micropores.

1.3.2. 10 million SBBs prepared in step 1.1 were suspended in 50 µL of alcohol and evenly spread on the microporous area of the chip. After standing for 10 minutes, the chip was incubated on a horizontal shaker for 30 minutes.

1.3.3. The chip was placed on a magnet and gently washed with 1×PBS to remove the unloaded beads. The loading rate of beads was required to be ≥95%; otherwise, steps 1.2.2 and 1.2.3 were repeated.

1.3.4. The chip was dried in a 60°C oven for 30 minutes and stored in a 4°C refrigerator for later use.

1.4. Nucleic acid sequence decoding

The fluorescent probe hybridization method described in the 2004 Genome Research article "Decoding Randomly Ordered DNA Arrays" was used to perform decoding to obtain the spatial nucleic acid sequences coupled to the surface of the beads in all the micropores of the chip.

1.5. Coupling of concanavalin A (ConA) which can bind to cell glycoproteins.

1.5.1. The Solulink Protein-Oligonucleotide Conjugation Kit or a similar kit was used to conjugate ConA protein with the nucleic acid sequence 5'P-tccactaccttctcctcctac-NH2-3', forming a nucleic acid-protein conjugate.

1.5.2. The decoded chip from step 1.3 was taken, and T4 ligase was used to couple the conjugate 5'P-tccactaccttctcctcctac-ConA to the spatial nucleic acid molecules 3' on the chip. The reaction system is shown in Table 1:

**Table 1: Reaction system for coupling ConA which can bind to cell glycoproteins.**

| Component | Volume/Sample |
|---|---|
| T4 Ligation Buffer | 20 µL |
| T4 DNA Ligase | 5 µL |
| 5'P -tccactaccttctcctcctac-ConA | 5 µL |
| Reverse sequence: | 5 µL |
| GTAGGAGGAGAAGGTAGTGGAAAAAAAAAAAAAA AAAAGCGTCT | |
| Nuclease-free water | 65 µL |
| Total | 100 µL |

1.5.3. The chip was cleansed and stored for later use.

### Example 2 Spatially Tagged WGA Chip for Single-Cell Transcriptome Library Construction from DSP-Fixed Tissue

2.1. Mouse liver tissue was fixed on ice with 1×DSP for 30 minutes and then embedded in OCT;
2.2 The tissue was sliced at a thickness of 40 µm and placed on the WGA chip prepared in Example 1.

2.3 The slices were treated with PBS containing 1% TritonX-100 for 30 minutes to permeabilize the tissue.

2.4 According to the NEB user manual, 100 µL of USER enzyme reaction solution was prepared and added to the tissue. The reaction was carried out at 37°C for 30 minutes to release the spatially tagged WGA, which then bound to the nearby tissue cells to achieve spatial tagging. Care was taken to control the humidity to prevent the reaction solution from drying out.

2.5 The chip was gently washed three times with 1×PBST to remove unbound free WGA.

2.6 The spatially tagged slices were dissociated into a single-cell suspension using the SeekMate^{®} Tissue Dissociation Reagent kit A, taking care to minimize cell loss during centrifugation.

2.7 According to the SeekOne^{®} DD Single-Cell 3' Transcriptome Kit, the cells obtained from step 2.4 were subjected to reverse transcription in a water-in-oil emulsion, and the cDNA products were purified from the oil phase.

2.8 The amplification mix was prepared according to Table 2.

The amplification system was added to 23 mL of purified cDNA product obtained according to the SeekOne^{®} DD Single-Cell 3' Transcriptome Kit user manual, mixed by pipetting up and down 10 times, and briefly centrifuged. PCR was then performed according to Step 2-2 of the instructions of the SeekOne^{®} DD Single-Cell 3' Transcriptome Kit. Note that additional spatial primers at fixed concentrations need to be added in addition to the standard reagent system.

**Table 2: Amplification mix**

| Component | Volume |
|---|---|
| 2×PCR Master Mix | 25 mL |
| cDNA Primers | 2 mL |
| Spatial primer (2.5 mM) | 0.4 mL |
| Primer sequence: | |
| CGTCCGTCGTTGCTCG | |
| Total | 27.4 mL |

### 2.9. PCR product purification

30 µL (0.6×) of DNA sorting magnetic beads were pipetted and added into the cDNA amplification product, mixed by pipetting up and down or oscillating 10 times, and then centrifuged briefly. The mixed product was left at room temperature for 5 minutes, then placed on a magnetic stand for absorption until the solution was clear, and all the supernatant was transferred to a new 0.2 mL PCR tube and stored at room temperature. A. The product obtained by purification of the magnetic beads after removal of the supernatant was used for 3' transcriptome library construction; and B. 40 µL of the supernatant was transferred to a new 0.2 mL PCR tube, and the product obtained from purification of the supernatant was used for sample tag library construction.

2.9-A. Purification of magnetic beads (for 3' transcriptome library construction)

2.9-A.1. The resulting product was kept on the magnetic stand, 200 µL of 80% ethanol was added, and the supernatant was carefully removed after about 30 seconds. The step was repeated once.

2.9-A.2. The resulting product was briefly centrifuged, and all residual supernatant was removed using a 10 µL pipette.

2.9-A.3. The resulting product was left at room temperature to allow the ethanol to completely evaporate (the magnetic beads appeared matte, about 3 minutes to 5 minutes), 40.5 µL of Nuclease-free Water was added to fully suspend the magnetic beads, and the resulting product was left at room temperature for 2 minutes.

2.9-A.4. The resulting product was placed on the magnetic stand for absorption until the solution was clear, and 40 µL of the supernatant was transferred to a new 0.2 mL PCR tube.

2.9-B. Purification of supernatant (for mixed sample tag library construction)

2.9-B.1. The DNA sorting magnetic beads were oscillated and mixed evenly, 35 µL (2×) of DNA sorting magnetic beads were pipetted and added into the supernatant, mixed by pipetting up and down or oscillating 10 times, and then centrifuged briefly.

2.9-B.2. The mixed product was left at room temperature for 5 minutes, then placed on a magnetic stand for absorption until the solution was clear, and the supernatant was removed.

2.9-B.3. The resulting product was kept on the magnetic stand, 200 µL of 80% ethanol was added, and the supernatant was carefully removed after about 30 seconds. The step was repeated once.

2.9-B.4. The resulting product was briefly centrifuged, and all residual supernatant was removed using a 10 µL pipette.

2.9-B.5. The resulting product was left at room temperature to allow the ethanol to completely evaporate (the magnetic beads appeared matte, about 3 minutes to 5 minutes), 20.5 µL of Nuclease-free Water was added to fully suspend the magnetic beads, and the resulting product was left at room temperature for 2 minutes.

2.9-B.6. The resulting product was placed on the magnetic stand until the solution was clear, and 20 µL of the supernatant was transferred to a new 0.2 mL PCR tube.

### 2.10. Construction of 3' transcriptome library

The purified cDNA product from step 2.12-A was used to construct the 3' transcriptome library according to the library construction in Step 3 of the instructions of the SeekOne^{®} DD Single Cell 3' Transcriptome kit.

### 2.11. Construction of sample tag library

2.11.1. Amplification of sample tag library

The mixed sample tag library was constructed using the purified DNA product from Step 2.9-B. A sample of Index PCR Mix was prepared according to the following table, and about 1 ng to 2 ng of the purified DNA product from Step 4-2B was added into the amplification system, and the mixture was mixed by pipetting up and down 10 times, and centrifuged briefly.

**Table 3 Sample of IndexPCR Mix**

| Component | Volume |
|---|---|
| 2×PCR Master Mix | 25 µL |
| N5 | 1 µL |
| N7 | 1 µL |
| ddH2O | To 50 µL |

The sample tag library and its 3' transcriptome library cannot use the same set of N5 and N7.

2.11.2. The PCR program was set and run according to Table 4.

**Table 4 PCR program parameters**

| Temperature | Time | Number of amplification cycles |
|---|---|---|
| 98°C | 3 minutes | |
| 98°C | 10 seconds | 13 cycles |
| 63°C | 15 seconds | |
| 72°C | 3 minutes | |
| 72°C | 5 minutes | |
| 4°C | hold | |

### 2.11.3. Purification of sample tag library

60 µL (1.2×) of DNA sorting magnetic beads were pipetted and added into the PCR product, mixed by pipetting up and down or oscillating 10 times, and then centrifuged briefly.

The mixed product was left at room temperature for 5 minutes, then placed on a magnetic stand for absorption until the solution was clear, and the supernatant was removed.

The resulting product was kept on the magnetic stand, 200 µL of 80% ethanol was added, and the supernatant was carefully removed after about 30 seconds. The step was repeated once.

The resulting product was briefly centrifuged, and all residual supernatant was removed using a 10 µL pipette.

The resulting product was left at room temperature to allow the ethanol to completely evaporate (the magnetic beads appeard matte, about 3 minutes to 5 minutes), 30.5 µL of Nuclease-free Water was added to fully suspend the magnetic beads, and the resulting product was left at room temperature for 2 minutes.

The resulting product was placed on the magnetic stand for absorption until the solution was clear, and 30 µL of the supernatant was transferred to a new 0.2 mL PCR tube.

### 2.11.4. Quality control of sample tag library

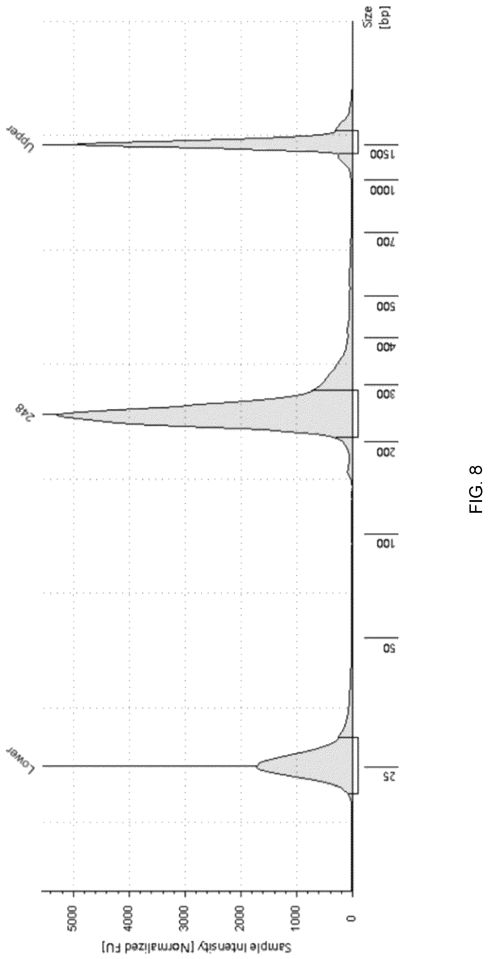

As shown in FIG. 8, the main peak size of the qualified library was from 200 bp to 350 bp (Agilent 4200 TapeStation) and the library concentration was >1 ng/µL (measured by Qubit 4.0).

### 2.11.5. High-throughput sequencing

The transcriptome library and the sample tag library were mixed in a ratio of 4:1, and high-throughput sequencing was performed using the Illumina sequencing platform and BGI sequencing platform, with a sequencing volume of ≥50000 reads/cell.

### Example 3 Single-cell transcriptome library construction by spatial tag nucleic acid chip for formaldehyde-fixed tissue

3.1. Mouse liver tissue was fixed with 4% paraformaldehyde at room temperature for 24 hours, and then embedded in OCT.

3.2 The tissue was sliced at a thickness of 40 µm and attached to the nucleic acid chips prepared in Example 1.3.

3.3 The tissue slices were permeabilized by being treated with PBS containing 1% TritonX-100 for 30 minutes.

3.4 100 µL of USER enzyme reaction solution was prepared according to the instruction of NEB and added dropwise to the tissue, and the reaction was carried out at 37°C for 30 minutes to release the spatial tags and bind them to the polyA tails of the mRNA in the tissue cells for spatial tagging. Humidity was controlled to prevent the reaction solution from drying.

3.5 The chips were gently washed 3 times with 1×PBST to remove unbound free spatially tagged nucleic acids.

3.6 The reverse transcription system was prepared using components from the SeekOne^{®} DD Single-Cell 3' Transcriptome Kit according to Table 5:

**Table 5 Reverse transcription system**

| Reagent | Volume/Sample |
|---|---|
| RT Buffer | 20.8 µL |
| Indicator | 8.0 µL |
| RT Enzyme | 5.2 µL |
| ddH₂O | 44.4 µL |
| Reducing Buffer | 1.6 µL |
| Total | 80 µL |

3.7 The prepared reverse transcription system was added dropwise to the tissue slices, incubated at 42°C for 30 minutes, and humidity was controlled to prevent the reaction solution from drying.

3.8 The spatially tagged slices were dissociated into single-cell suspensions using the SeekMate^{®} Tissue Dissociation Kit A. Cell loss during centrifugation was minimized.

3.9 The water-in-oil ligation of the single cells obtained in step 3.8 was performed using the water-in-oil reagents and methods from step two of Example 1 of patent application No. CN202210874581.6. At this point, the cell tags were ligated to the 5' end of the spatial spatial tags.

### 3.10 Demulsification and decrosslinking

3.10.1 After the water-in-oil ligation reaction, 100 µL of Demulsion Agent reagent was added to the PCR tube, allowed to stand at room temperature for 2 minutes, and briefly centrifuged.

3.10.2 120 µL of the clear oil phase was slowly aspirated from the bottom of the PCR tube and discarded without aspirating the upper aqueous phase. The fixed cells were now in the upper aqueous phase.

3.10.3 The upper aqueous phase solution was transferred to a 1.5 ml centrifuge tube and centrifuged at 1000g for 5 minutes at 4°C. The supernatant was carefully removed. 1 ml of 1x PBS containing 0.1% (v/v) Triton x-100 was added, centrifuged again at 1000g for 5 minutes at 4°C, and the supernatant was completely removed.

3.10.4 Proteinase K and 2% (v/v) SDS were added to the tube and incubated at 55°C for 1 hour for the decrosslinking reaction.

3.10.5 After the reaction, an equal volume of DNA clean beads was added for purification and eluted with 30.5 µL of double-distilled water.

3.10.6 30 µL of the supernatant was transferred to a new 0.2 mL centrifuge tube.

### 3.11 Template switching

The template switching system was prepared using components from the SeekOne^{®} DD Single-Cell 3' Transcriptome Kit according to Table 6:

**Table 6 Template switching system**

| Reagent | Volume/Sample |
|---|---|
| RT Buffer | 20.9 µL |
| Indicator | 8.0 µL |
| RT Enzyme | 5.2 µL |
| ddH₂O | 12.3 µL |
| TSO | 2.0 µL |
| Reducing Buffer | 1.6 µL |
| Total | 50 µL |

3.12 The prepared template switching system was added to the purified product from step 3.10.6, reacted at 42°C for 90 minutes, and then inactivated at 85°C for 5 minutes.

3.13 The sample was purified with 1× sorting beads and eluted with 23.5 µL of double-distilled water.

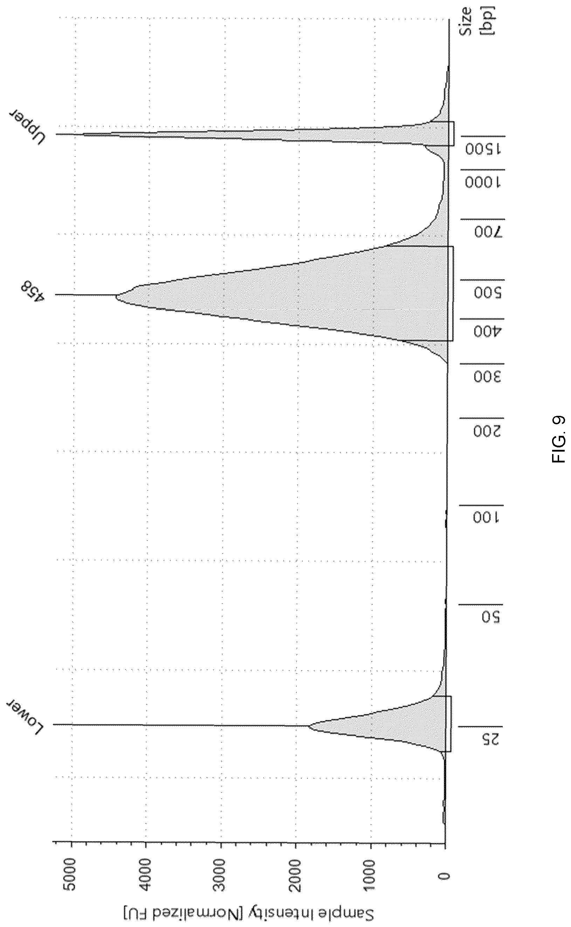

3.14 The purified cDNA product was amplified and purified according to the instructions of the SeekOne^{®} DD Single-Cell 3' Transcriptome Kit, and the 3' transcriptome library was constructed. The final library is shown in Figure 9, with the main peak of the library fragment being 458 bp, without any smearing bands.

### 3.15 High-throughput sequencing

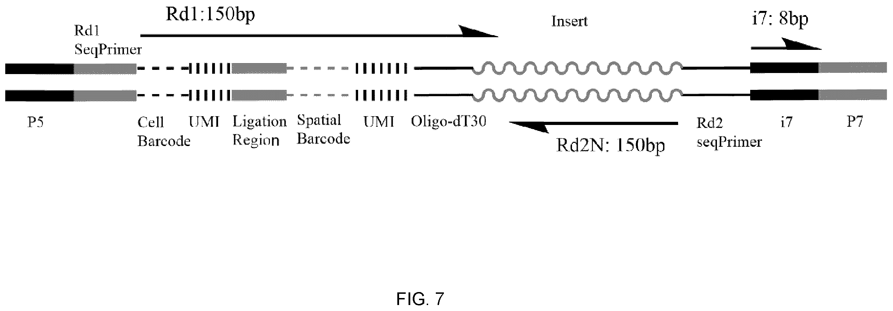

The constructed library has a structure as shown in Figure 7, and high-throughput sequencing was performed on the Illumina sequencing platform and the BGI sequencing platform using the PE150 strategy.

In conclusion, the present disclosure provides a method for constructing a single-cell library containing spatial information of cells in tissue. By contacting the nucleic acid chips having spatial information with slices of a tissue to be tested, the spatial information can be released into the tissue cells, so that during the subsequent dissociation of the tissue cells, the spatial information of the tissue can be read, thereby enabling the analysis of genetic information at the single-cell level while also obtaining the spatial information of the single cells.

The above are only preferred examples of the present disclosure and are not intended to limit the scope of the present disclosure. Various modifications and changes may be made to the present disclosure by a person skilled in the art. Any modifications, equivalent replacements, improvements, etc., made within the spirit and principles of the present disclosure should be included within the protection scope of the invention.

## Claims

1. A method for constructing a single-cell library containing spatial information of cells in tissue, **characterized by** comprising steps of:
S10: providing a chip with multiple sites, each of the sites having nucleic acid molecules with a known base sequence, wherein the nucleic acid molecules contain spatial information;
S20: contacting a slice of a tissue to be tested with the chip so that the nucleic acid molecules with known base sequences in the chip bind to cells of the slice of the tissue to be tested, resulting in a bound tissue slice;
S30: dissociating the bound tissue slice to prepare a single-cell suspension;
S40: constructing a library for the single-cell suspension using a high-throughput single-cell library construction and sequencing platform to obtain a transcriptome library and a spatial tag library; and
S50: sequencing the transcriptome library and the spatial tag library to obtain the single-cell library containing spatial information of cells in tissue.

2. The method for constructing a single-cell library containing spatial information of cells in tissue according to claim 1, **characterized in that**, in step S10,
nucleic acid molecules at different sites have different known base sequences, and nucleic acid molecules at the same site have the same known base sequence; and/or
a spacing between sites is 100 nm to 1 mm.

3. The method for constructing a single-cell library containing spatial information of cells in tissue according to claim 1, **characterized in that**, in step S10,
the chip is planar, and the nucleic acid molecules with spatial information are fixed onto a surface of the chip in relatively independent clusters;
the chip has micropores in which a medium is contained, and the nucleic acid molecules with spatial information are distributed on the medium in the micropores of the chip or coupled to a surface of the chip;
the chip has micropores in which a medium is contained, and the medium in the micropores of the chip comprises any one of polymer beads, magnetic beads, and hydrogel beads; and/or
a material of the chip comprises any one of glass, ITO glass, polymer, ITO polymer, and silicon wafer.

4. The method for constructing a single-cell library containing spatial information of cells in tissue according to claim 1, **characterized in that**, in step S10,
the nucleic acid molecule with spatial information is coupled with a molecule that binds to a specific substance in the cell.

5. The method for constructing a single-cell library containing spatial information of cells in tissue according to claim 4, **characterized in that**, the molecule that binds to the specific substance in the cell comprises a lectin, and the specific substance in the cell is a glycoprotein;
the molecule that binds to the specific substance in the cell comprises an antibody, and the specific substance in the cell is β2-microglobulin;
the molecule that binds to the specific substance in the cell comprises a lipid molecule, and the specific substance in the cell is a lipid bilayer; or
the molecule that binds to the specific substance in the cell comprises a nucleic acid, and the specific substance in the cell is mRNA.

6. The method for constructing a single-cell library containing spatial information of cells in tissue according to claim 1, **characterized in that**, between step S10 and step S20, the method further comprises:
soaking the cells of the slice of the tissue to be tested with any one of fixatives selected from an aldehyde fixative solution, an acid fixative solution, an alcohol fixative solution, and a DSP crosslinker; and/or
soaking the cells of the slice of the tissue to be tested with an alcohol or a surfactant.

7. The method for constructing a single-cell library containing spatial information of cells in tissue according to claim 1, **characterized in that**, in step S10, the chip is prepared by steps of:
S01: synthesizing various nucleic acid molecules with different spatial tag sequences; and
S02: contacting the synthesized nucleic acid molecules with corresponding positions on the chip, so that the synthesized nucleic acid molecules are fixed to the corresponding positions on the chip, to obtain the chip with the nucleic acid molecules containing spatial information.

8. The method for constructing a single-cell library containing spatial information of cells in tissue according to claim 7, **characterized in that**, in step S01,
the nucleic acid molecule comprises a fixed sequence -a conditionally cleavable site - a spatial tag - a cell binding molecule, wherein the conditionally cleavable site comprises any one of a disulfide modification, a dU modification, an RNA base modification, a dI modification, a DSpacer modification, an AP site modification, a photocleavable (PC) linker, and a restriction enzyme recognition sequence; and/or
the nucleic acid molecule comprises a 5' coupling group and a molecular tag sequence, wherein, the 5' coupling group comprises any one of a chemical crosslinker, biotin, avidin, and streptavidin.

9. The method for constructing a single-cell library containing spatial information of cells in tissue according to claim 7, **characterized in that**, in step S01,
the nucleic acid molecule comprises a 5' coupling group and a molecular tag sequence, wherein the 5' coupling group comprises any one of a chemical crosslinker, biotin, avidin, and streptavidin; and the chemical crosslinker comprises any one of cystamine, glutaraldehyde, S,S-dimethyl sulfoximine, N-hydroxysulfosuccinimide crosslinker BS3, formaldehyde, carbodiimide, SMCC, sulfo-SMCC, vinylsilane, N,N'-diallyltartaridiamide, and N,N'-bis(acryloyl)cystamine.

10. The method for constructing a single-cell library containing spatial information of cells in tissue according to claim 7, **characterized in that**, in step S02,
a method for fixing the synthesized nucleic acid molecules to the corresponding positions on the chip comprises either of an in situ contact method and a random fixation method.

11. A sequencing method, **characterized by** comprising steps of:
constructing a single-cell library using the method for constructing a single-cell library containing spatial information of cells in tissue according to any one of claims 1 to 10, to obtain a transcriptome library and a spatial tag library;
sequencing the obtained transcriptome library and the spatial tag library to obtain single-cell transcriptome data and spatial tag library data; and
mapping spatial information and genetic information of the nucleic acid molecules with known base sequences to the tissue to be tested to obtain spatial information and genetic information of single cells in the tissue to be tested.
